(19) Europäisches Patentamt
European Patent Office
Office européen des brevets

(11) Veröffentlichungsnummer : **0 085 652**
**B1**

(12) # EUROPÄISCHE PATENTSCHRIFT

(45) Veröffentlichungstag der Patentschrift : **13.03.85**

(51) Int. Cl.⁴ : **C 07 C121/45**, C 07 C121/48

(21) Anmeldenummer : **83810028.7**

(22) Anmeldetag : **24.01.83**

(54) **1,10-substituierte 10-Amino-deca-3,7-dien-nitrile und Verfahren zu deren Herstellung.**

(30) Priorität : **29.01.82 CH 559/82**

(43) Veröffentlichungstag der Anmeldung : **10.08.83 Patentblatt 83/32**

(45) Bekanntmachung des Hinweises auf die Patenterteilung : **13.03.85 Patentblatt 85/11**

(84) Benannte Vertragsstaaten : **CH DE FR GB IT LI NL**

(56) Entgegenhaltungen :
**Keine Entgegenhaltungen.**
**Keine**

(73) Patentinhaber : **CIBA-GEIGY AG**
**Postfach**
**CH-4002 Basel (CH)**

(72) Erfinder : **Reinehr, Dieter, Dr.**
**Wolfsheule 10**
**D-7842 Kandern (DE)**
Erfinder : **Pfeifer, Josef, Dr.**
**Brunnmattstrasse 32**
**CH-4106 Therwil (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Gegenstand der Erfindung sind neue 1,10-substituierte 10-Amino-deca-3,7-dien-nitrile sowie ein Verfahren zu deren Herstellung. Die neuen 10-Amino-deca-3,7-dien-nitrile stellen wertvolle Ausgangsprodukte zur Herstellung von entsprechenden 1,11-Diamino-undeca-3,7-dienen dar, die ihrerseits z. B. Anwendung als Polykondensationskomponenten, vor allem zur Herstellung von vernetzbaren Polyamiden, finden.

Die neuen 1,10-substituierten 10-Amino-deca-3,7-dien-nitrile entsprechen der Formel I

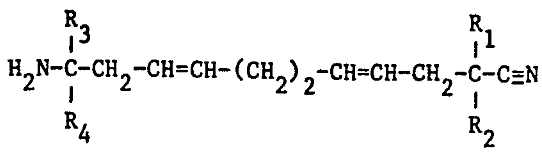

(I)

worin

$R_1$ Alkyl mit 1-12 C-Atomen,

$R_2$ Wasserstoff oder Alkyl mit 1-12 C-Atomen,

$R_3$ Alkyl mit 1-12 C-Atomen, Cycloalkyl mit 4-12 Ring-C-Atomen, Aralkyl mit 7 oder 8 C-Atomen, gegebenenfalls substituiertes Aryl, oder, wenn $R_4$ Wasserstoff ist, auch —CH = CH-Alkyl oder —C(Alkyl) = CH-Alkyl mit je 1-4 C-Atomen in den Alkylteilen und

$R_4$ Wasserstoff, Alkyl mit 1-12 C-Atomen, Cycloalkyl mit 4-12 Ring-C-Atomen, Aralkyl mit 7 oder 8 C-Atomen oder gegebenenfalls substituiertes Aryl darstellen oder

$R_1$ und $R_2$ und/oder $R_3$ und $R_4$ zusammen Alkylen mit 3-11 C-Atomen bedeuten.

Durch $R_1$ bis $R_4$ dargestellte Alkylgruppen können geradkettig oder verzweigt sein. Alkylgruppen $R_1$, $R_2$ und $R_4$ weisen bevorzugt 1-5 C-Atome auf und sind geradkettig. Alkylgruppen $R_3$ weisen mit Vorteil 1-7 C-Atome und insbesondere 1-5 C-Atome auf. Beispiele von Alkylgruppen $R_1$ bis $R_4$ sind : die Methyl-, Aethyl-, n-Propyl-, Isopropyl-n,-, sek- und tert-Butyl-, n-Pentyl-, 2- oder 3-Pentyl-, n-Hexyl-, 2- oder 3-Heptyl-, n-Octyl-, n-Decyl- und n-Dodecylgruppe.

Stellt $R_3$ eine Gruppe —CH = CH-Alkyl oder —C(Alkyl) = CH-Alkyl dar, so sind die Alkylgruppen in diesen Substituenten bevorzugt geradkettig und bedeuten insbesondere Methyl oder Aethyl.

Cycloalkylgruppen $R_3$ und $R_4$ können unsubstituiert oder durch $C_{1-4}$-Alkylgruppen substituiert sein. Insbesondere handelt es sich dabei um durch eine Methyl- oder Aethylgruppe substituiertes Cycloalkyl.

Bevorzugt sind Cycloalkylgruppen $R_3$ und $R_4$ jedoch unsubstituiert und weisen 5-8 Ring-C-Atome auf. Besonders bevorzugt sind die Cyclopentyl- und vor allem die Cyclohexylgruppe.

Als Aralkylgruppen $R_3$ und $R_4$ kommen vor allem die Benzyl-, Methylbenzyl- oder Phenyläthylgruppe in Betracht. Stellt $R_3$ oder $R_4$ substituiertes Aryl dar, so kommen als Substituenten vor allem Alkylgruppen mit 1-4 und insbesondere 1 oder 2 C-Atomen in Betracht. Arylgruppen $R_3$ und $R_4$ können mehrere Alkylgruppen tragen, sind aber bevorzugt nur durch eine Alkylgruppe substituiert. Besonders bevorzugt sind die 1- oder 2-Naphthylgruppe, durch eine Alkylgruppe mit 1-4 und besonders 1 oder 2 C-Atomen substituiertes Phenyl und ganz besonders unsubstituiertes Phenyl.

Durch $R_1$ und $R_2$ und/oder $R_3$ und $R_4$ zusammen dargestellte Alkylengruppen weisen bevorzugt 4-7 C-Atome auf. Insbesondere handelt es sich dabei um die Tetramethylen- und ganz besonders die Pentamethylengruppe.

Bevorzugt sind Verbindungen der Formel I, worin $R_1$ Alkyl mit 1-5 C-Atomen, $R_2$ Wasserstoff oder Alkyl mit 1-5 C-Atomen oder $R_1$ und $R_2$ zusammen Alkylen mit 4-7 C-Atomen, $R_3$ Alkyl mit 1-7 C-Atomen, Cycloalkyl mit 5-8 C-Atomen oder unsubstituiertes Phenyl oder, bei $R_4$ = H, auch —$C(C_2H_5)$ = $CHCH_3$ und $R_4$ Wasserstoff oder Alkyl mit 1-5 C-Atomen bedeuten. Besonders bevorzugt sind Verbindungen der Formel I, worin $R_1$ Alkyl mit 1-5 C-Atomen, $R_2$ Wasserstoff oder Alkyl mit 1-5 C-Atomen oder $R_1$ und $R_2$ zusammen Alkylen mit 4-7 C-Atomen, $R_3$ Alkyl mit 1-5 C-Atomen, unsubstituiertes Phenyl oder, bei $R_4$ = H, —$C(C_2H_5)$ = $CHCH_3$ und $R_4$ Wasserstoff oder Methyl bedeuten, vor allem Verbindungen der Formel I, worin $R_1$ Methyl oder Aethyl, $R_2$ Wasserstoff, Methyl oder Aethyl, $R_3$ Alkyl mit 1-5 C-Atomen oder unsubstituiertes Phenyl und $R_4$ Wasserstoff oder Methyl bedeuten. Ganz besonders bevorzugt ist die Verbindung der Formel I, worin $R_1$ und $R_2$ Methyl, $R_3$ Isopropyl und $R_4$ Wasserstoff darstellen.

Als Beispiele spezifischer Verbindungen der Formel I seien genannt :

1,1-Dimethyl-10-isopropyl-10-amino-deca-3,7-dien-nitril,
1,1-Dimethyl-10-methyl-10-amino-deca-3,7-dien-nitril,
1,1-Dimethyl-10-äthyl-10-amino-deca-3,7-dien-nitril,
1,1-Dimethyl-10-n-hexyl-amino-deca-3,7-dien-nitril,
1,1-Dimethyl-10-phenyl-amino-deca-3,7-dien-nitril,
1,1-Dimethyl-10-cyclohexyl-amino-deca-3,7-dien-nitril,
1,1-Diäthyl-10-isopropyl-amino-deca-3,7-dien-nitril,
1,1-Diäthyl-10-(3-pentyl)-amino-deca-3,7-dien-nitril,

1,1-Dimethyl-10-pentamethylen-amino-deca-3,7-dien-nitril,
1,1-Diäthyl-10-phenyl-amino-deca-3,7-dien-nitril,
1-Methyl, 10-phenyl-amino-deca-3,7-dien-nitril,
1-Methyl-1-n-propyl-10-(2-pentyl)-amino-deca-3,7-dien-nitril,
1-Methyl-10-cyclohexyl-amino-deca-3,7-dien-nitril,
1-Methyl-10-(3-pentyl)-amino-deca-3,7-dien-nitril,
1-Aethyl-1-n-butyl-10-(3-heptyl)-amino-deca-3,7-dien-nitril,
1-Aethyl-1-n-butyl-10-äthyl-amino-deca-3,7-dien-nitril,
1-Aethyl-1-n-butyl-10-phenyl-amino-deca-3,7-dien-nitril,
1,10,10-Trimethyl-amino-deca-3,7-dien-nitril,
1-Pentamethylen-10-cyclohexyl-amino-deca-3,7-dien-nitril,
1-Tetramethylen-10-cyclopentyl-amino-deca-3,7-dien-nitril,
1,1,10,10-Tetramethyl-amino-deca-3,7-dien-nitril.

Die Verbindungen der Formel I können dadurch hergestellt werden, dass man Verbindungen der Formel II

$$H_2N-\underset{R_4}{\overset{R_3}{C}}-CH_2-CH=CH-(CH_2)_2-CH=CH-CH_2-\underset{R_2}{\overset{R_1}{C}}-CH=NOH \qquad (II)$$

worin $R_1$, $R_2$, $R_3$ und $R_4$ die unter Formel I angegebene Bedeutung haben, dehydratisiert.

Die Dehydratisierung der Verbindungen der Formel II kann auf an sich bekannte Weise chemisch oder thermisch vorgenommen werden.

Als Dehydratisierungsmittel für die chemische Dehydratisierung eignen sich z. B. Anhydride von gegebenenfalls durch Halogenatome oder Alkylgruppen substituierten aliphatischen Monocarbonsäuren mit 2-5 C-Atomen, wie Essigsäure-, Propionsäure-, Buttersäure- und Valeriansäureanhydrid, Trichlor-, Trifluor-, Trimethyl-, Triäthyl- und Tri-n-butylessigsäureanhydrid ; Alkylester mit 1-5 C-Atomen der Chlorameisensäure, wie der Chlorameisensäuremethyl-, -äthyl-, -isopropyl- und -isobutylester ; Acetylchlorid, Thionylchlorid, Benzoylchlorid, Benzolsulfonylchlorid, Sulfurylchlorid, Phosphorpentoxid im Gemisch mit Aethanol, Phosphoroxichlorid, Polyphosphorsäure und wässriges Alkali, wie wässriges NaOH oder KOH. Die genannten Dehydratisierungsmittel können gegebenenfalls im Gemisch mit Katalysatoren, z. B. Erdalkalimetall- oder Alkalimetallsalzen von aromatischen Monocarbonsäuren oder von aliphatischen Monocarbonsäuren mit 1-3 C-Atomen, wie Natriumbenzoat, Natriumsalicylat, Calcium- und Natriumformiat, Calcium-, Magnesium-, Natrium- und Kaliumacetat und Natriumpropionat, oder tertiären Basen, wie Triäthylamin, Pyridin und Dimethylanilin, eingesetzt werden.

Bevorzugtes Dehydratisierungsmittel ist Essigsäureanhydrid. Die chemische Dehydratisierung wird mit Vorteil in Gegenwart eines unter den Reaktionsbedingungen inerten organischen Verdünnungs- oder Lösungsmittel durchgeführt. Geeignete Verdünnungs- bzw. Lösungsmittels sind z. B. aromatische Kohlenwasserstoffe, wie Benzol und Toluol, aromatische oder aliphatische Nitrile, besonders Benzonitril und Alkylnitrile mit 2-5 C-Atomen, wie Acetonitril, Propionitril und Butyronitril, sowie wasserfreie Essigsäure. Bevorzugte Verdünnungs- bzw. Lösungsmittel sind Acetonitril und vor allem wasserfreie Essigsäure.

Die chemische Dehydratisierung wird im allgemeinen bei Temperaturen zwischen 50 °C und der Rückflusstemperatur des Reaktionsgemisches vorgenommen.

Die thermische Dehydratisierung kann z. B. über Aluminiumoxid oder Thoriumoxid bei Temperaturen zwischen 340 und 360 °C durchgeführt werden. Die chemische Dehydratisierung ist bevorzugt.

Die Ausgangsprodukte der Formel II sind bekannt und können nach dem in dem europäischen Patent Nr. 11599 beschriebenen Verfahren hergestellt werden.

Die Verbindungen der Formel I können z. B. durch Reduktion in entsprechend substituierte 1,11-Diamino-undeca-3,7-diene der Formel III

$$H_2N-\underset{R_4}{\overset{R_3}{C}}-CH_2-CH=CH-(CH_2)_2-CH=CH-CH_2-\underset{R_2}{\overset{R_1}{C}}-CH_2NH_2 \qquad (III)$$

übergeführt werden, z. B. mittels Bouveault-Blanc-Reduktion mit Natriummetall in Alkoholen, bevorzugt Isopropanol. In Formel III haben $R_1$ bis $R_4$ die unter Formel I angegebene Bedeutung.

Die Diamine der Formel III sind wertvolle Zwischenprodukte und finden z. B. Anwendung als Polykondensationskomponenten zur Herstellung von transparenten vernetzbaren Polyamiden. Solche Polyamide können auf an sich bekannte Weise durch Polykondensation von Diaminen der Formel III mit

gesättigten oder ungesättigten aliphatischen und/oder aromatischen Dicarbonsäuren oder amidbildenden Derivaten davon, vorzugsweise in Gegenwart einer anorganischen oder organischen Phosphorverbindung als Beschleuniger, hergestellt werden. Sie können thermisch oder photochemisch vernetzt werden, bevorzugt in Gegenwart von radikalischen Initiatoren. Die photochemische Vernetzung erfolgt mit Vorteil durch Polythiole mit mindestens zwei Thiolgruppen pro Molekül in Gegenwart von Photosensibilisatoren, wie Benzophenon, Thioxanthon und dergl. Die genannten Polyamide bzw. deren Gemische mit Polythiolen und Sensibilisatoren eignen sich z. B. zur Herstellung von lösungsmittelbeständigen Ueberzügen auf verschiedenen Materialien, besonders Metallen, zur Bilderzeugung unter Lichteinwirkung oder zur Herstellung von transparenten Formkörpern, z. B. nach dem Spritzguss- oder Extrusionsverfahren. Die unter Verwendung von Diaminen der Formel III und aromatischen und/oder aliphatischen Dicarbonsäuren, besonders Terephthalsäure, Isophthalsäure und/oder Adipinsäure, erhaltenen transparenten Polyamide zeichnen sich durch geringe Wasseraufnahme, hohe Hydrolysebeständigkeit und/oder gute Dimensionsstabilität unter Feuchtigkeitseinwirkung aus. Sie ergeben ferner sehr gut haftende, lösungsmittelbeständige Ueberzüge und weisen im Gegensatz zu vorbekannten ungesättigten Polyamiden eine gute Verträglichkeit mit Polythiolen auf. Dank ihrer guten Löslichkeit in verschiedenen organischen Lösungsmitteln lassen sie sich auch leicht thermisch vernetzen.

A) Herstellungsbeispiele

Beispiel 1

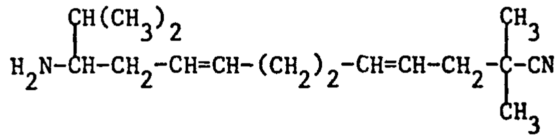

80 g (0,3 Mol) 2,2-Dimethyl-11-isopropyl-11-amino-undeca-4,8-dienaloxim werden unter Rühren mit 100 ml Eisessig versetzt. Dann wird HCl-Gas bis zur Sättigung eingeleitet, und innerhalb von 15 Minuten werden 30,6 g (0,3 Mol) Essigsäureanhydrid zugetropft. Man erhitzt die Reaktionsmischung noch 4 Stunden unter Rückfluss, destilliert den Eisessig ab und löst den Rückstand in Wasser. Nachdem man die Lösung mit Natronlauge basisch gestellt hat, nimmt man die sich abscheidende organische Phase mit Toluol auf und destilliert. Man erhält 68,5 g (0,276 Mol) 1,1-Dimethyl-10-isopropyl-10-amino-deca-3,7-dien-nitril, entsprechend einer Ausbeute von 92 % d. Th. ; Sdp. 94 °C/3 Pa.

Analyse für $C_{16}H_{28}N_2$ (Molgewicht 248,41) :
berechnet : C 77,36 % H 11,36 % N 11,28 %
gefunden : C 77,25 % H 11,40 % N 10,98 %.

$^1$H-NMR-Spektrum τ(ppm) : 4,5 (m), 7,45 (quin), 7,9 (m), 2,0-2,5 (m), 8,65 (s), 8,84 (s), 9,07 (dd) im Verhältnis 4 : 1 : 8 : 1 : 6 : 2 : 6.

Beispiel 2

Es wird wie in Beispiel 1 beschrieben vorgegangen, jedoch unter Verwendung von 50,5 g (0,2 Mol) 2,2-Dimethyl-11-äthyl-11-amino-undeca-4,8-dienal-oxim, 25 g (0,245 Mol) Essigsäureanhydrid und 100 ml Acetonitril als Lösungsmittel. Nach der Aufarbeitung erhält man 37 g (0,159 Mol) 1,1-Dimethyl-10-äthyl-10-amino-deca-3,7-dien-nitril, entsprechend einer Ausbeute von 79,5 % d. Th. ; Sdp. 84 °C/1 Pa ; $n_D^{20}$ = 1,471 1.

Analyse für $C_{15}H_{26}N_2$ (Molgewicht 234, 39) :
berechnet : C 76,87 % H 11,18 % N 11,96 %
gefunden : C 76,88 % H 10,98 % N 11,82 %.

$^1$H-NMR-Spektrum τ(ppm) : 4,5 (m), 7,3 (m), 7,8 (m), 8,3-8,75 (m), 8,82 (s), 9,03 (t) im Verhältnis von 4 : 1 : 8 : 8 : 2 : 3.
MS-Spektrum : Molekülpeak 234, Bruchstückmassen 205, 166, 82.

Beispiel 3

Es wird wie in Beispiel 1 beschrieben vorgegangen, jedoch unter Verwendung von 473 g (1,65 Mol) 2-Methyl-11-phenyl-11-amino-undeca-4,8-dienal-oxim, 169 g (1,65 Mol) Essigsäureanhydrid, 250 ml Essigsäure und HCl-Gas im Ueberschuss. Nach der Aufarbeitung erhält man 160 g (0,6 Mol) 1-Methyl-10-phenyl-10-amino-deca-3,7-dien-nitril, entsprechend einer Ausbeute von 36,4 % d. Th. ; Sdp. 135 °C/1 Pa ; $n_D^{20}$ = 1,526 8.

Analyse für $C_{18}H_{24}N_2$ (Molgewicht 268,40) :
berechnet : C 80,55 % H 9,01 % N 10,44 %
gefunden : C 79,89 % H 8,90 % N 10,19 %.

$^1$H-NMR-Spektrum τ(ppm) : 2,72 (s), 4,55 (m), 6,09 (t), 7,4 (m), 7,6-8,0 (m), 8,33 (s), 8,72 (d) im Verhältnis von 5 : 4 : 1 : 1 : 8 : 2 : 3.
MS-Spektrum : Molekülpeak 268, Bruchstückmassen 214, 146, 106, 79.

Beispiel 4

Es wird wie in Beispiel 1 beschrieben vorgegangen, jedoch unter Verwendung von 148 g (0,62 Mol) 2,11,11-Trimethyl-11-amino-undeca-4,8-dienal-oxim, 63,3 g (0,62 Mol) Acetanhydrid, 250 ml Eisessig und HCl-Gas im Ueberschuss. Nach der Aufarbeitung erhält man 87 g (0,396 Mol) 1,10,10-Trimethyl-10-amino-deca-3,7-dien-nitril, entsprechend einer Ausbeute von 63,7 % d. Th. ; Sdp. 94 °C/4 Pa ; $n_D^{20}$ = 1,470 6.

Analyse für $C_{14}H_{24}N_2$ (Molgewicht 220,36) :
berechnet : C 76,31 % H 10,98 % N 12,72 %
gefunden : C 75,24 % H 11,05 % N 11,73 %.

$^1$H-NMR-Spektrum τ(ppm) : 4,5 (m), 7,35 (sex), 7,6-8,1 (m), 8,38 (s), 8,68 (d), 8,88 (s) im Verhältnis von 4 : 1 : 8 : 2 : 3 : 6.
MS-Spektrum : Molekülpeak 220, Bruchstückmassen 205, 190, 178, 98, 58.

Beispiel 5

Es wird wie in Beispiel 1 beschrieben vorgegangen, jedoch unter Verwendung von 150 g (0,59 Mol) 2,2,11,11-Tetramethyl-11-amino-undeca-4,8-dienal-oxim, 75 g (0,735 Mol) Acetanhydrid und 250 ml Eisessig. Nach der Aufarbeitung erhält man 135 g (0,576 Mol) 1,1,10,10-Tetramethyl-10-amino-deca 3,7-dien-nitril, entsprechend einer Ausbeute von 97,6 % d. Th. ; Sdp. 85 °C/1 Pa.

Beispiel 6

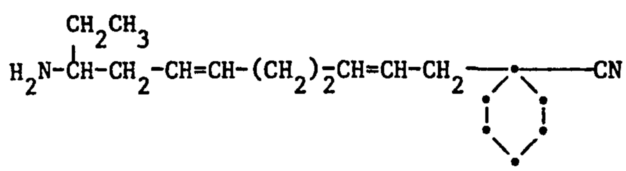

Es wird wie in Beispiel 1 beschrieben vorgegangen, jedoch unter Verwendung von 332 g (1,13 Mol) 2-Pentamethylen-11-äthyl-11-amino-undeca-4,8-dienal-oxim, 153 g (1,5 Mol) Acetanhydrid, 90 g Eisessig und HCl-Gas im Ueberschuss. Nach der Aufarbeitung erhält man 229 g (0,833 Mol) 1-Pentamethylen-10-äthyl-10-amino-deca-3,7-dien-nitril, entsprechend einer Ausbeute von 73,8 % d. Th. ; Sdp. 138 °C/1 Pa ; $n_D^{20}$ = 1,493 1.

Analyse für $C_{18}H_{30}N_2$ (Molgewicht 274,45) :
berechnet : C 78,77 % H 11,02 % N 10,21 %
gefunden : C 78,67 % H 10,83 % N 9,98 %.

$^1$H-NMR-Spektrum τ(ppm) : 4,6 (m), 7,4 (m), 7,8-8,9 (m), 9,1 (t) im Verhältnis 4 : 1 : 22 : 3.
MS-Spektrum : Molekülpeak 274, Bruchstückmassen 259, 245, 191, 166, 112, 98.

Beispiel 7

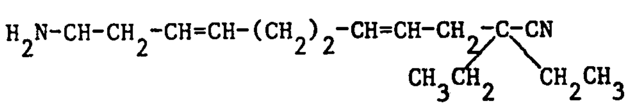

Es wird wie in Beispiel 1 beschrieben vorgegangen, jedoch unter Verwendung von 280 g (0,87 Mol) 2,2-Diäthyl-11-(3-pentyl)-11-amino-undeca-4,8-dienal-oxim, 112 g (1,2 Mol) Acetanhydrid, 75 g Eisessig und HCl-Gas im Ueberschuss. Nach der Aufarbeitung erhält man 138 g (0,455 Mol) 1,1-Diäthyl-10-(3-pentyl)-10-amino-deca-3,7-dien-nitril, entsprechend einer Ausbeute von 52,3 % d. Th., Sdp. 139 °C/3 Pa ; $n_D^{20}$ = 1,479 3.

Analyse für $C_{20}H_{36}N_2$ (Molgewicht 304,52)
berechnet : C 78,88 % H 11,92 % N 9,20 %
gefunden : C 78,90 % H 11,63 % N 9,45 %.

$^1$H-NMR-Spektrum τ(ppm) : 4,6 (m), 7,3 (m), 7,7-8,2 (m), 8,4-9,2 (m) im Verhältnis von 4 : 1 : 8 : 23.
MS-Spektrum-Molekülpeak 303, Bruchstückmassen 275, 233, 142, 100, 82.

B) Verwendungsbeispiele

## Beispiel I

Herstellung von 1,11-Diamino-undeca-3,7-dienen

23 g (1 Mol) Natrium werden zu 150 ml Toluol gegeben, und die Mischung wird bis zum Schmelzen des Natriums aufgeheizt. Dann wird die Heizung entfernt und solange gerührt, bis das Natrium als feingraue Dispersion zerteilt ist. Zu dieser Mischung tropft man dann eine Lösung von 53 g (0,214 Mol) 1,1-Dimethyl-10-isopropyl-10-amino-deca-3,7-dien-nitril in 100 ml Isopropanol. Es wird noch 3 Stunden unter Rückfluss gekocht, mit 200 ml Wasser versetzt, und die organische Phase wird abgetrennt. Nach dem Abdestillieren des Lösungsmittels erhält man 44 g (0,175 Mol) 2,2 Dimethyl-11-isopropyl-1,11-diamino-undeca-4,8-dien ; Spd. 86 °C/1 Pa ; $n_D^{20}$ = 1,481 0.

Auf analoge Weise werden hergestellt :

— 2,2-Dimethyl-11-äthyl-1,11-diamino-undeca-4,8-dien (Sdp. 80 °C/1 Pa ; $n_D^{20}$ = 1,480 9), unter Verwendung von 30 g (0,128 Mol) 1,1-Dimethyl-10-äthyl-10-amino-deca-3,7-dien-nitril, 100 ml Isopropanol, 23 g Natrium und 100 ml Xylol ;

— 2-Methyl-11-phenyl-1,11-diamino-undeca-4,8-dien (Spd. 103 °C/13 Pa ; $n_D^{20}$ = 1,529 3), unter Verwendung von 134 g (0,5 Mol) 1-Methyl-10-phenyl-10-amino-deca-3,7-dien-nitril, 58,5 g Natrium, 500 ml Toluol und 250 ml Isopropanol ;

— 2,11,11-Trimethyl-1,11-diamino-undeca-4,8-dien (Sdp. 80 °C/2 Pa ; $n_D^{20}$ = 1,478 5), unter Verwendung von 83 g (0,378 Mol) 1,10,10-Trimethyl-10-amino-deca-3,7-dien-nitril, 60 g Natrium, 500 ml Toluol und 250 ml Isopropanol ;

— 2,2,11,11-Tetramethyl-1,10-diamino-undeca-4,8-dien (Sdp. 100 °C/4 Pa ; $n_D^{20}$ = 1,477 2), unter Verwendung von 50 g (0,213 Mol) 1,1,10,10-Tetramethyl-10-amino-deca-3,7-dien-nitril, 95 g Natrium, 500 ml Toluol und 300 ml Isopropanol.

## Beispiel II

62,8 g 2,2-Dimethyl-11-isopropyl-1,11-diamino-undeca-4,8-dien, 36,4 g Adipinsäure, 0,25 ml einer 10 %igen wässrigen $NH_4H_2PO_2$-Lösung und 0,5 g Di-tert-butyl-p-kresol werden in einem Autoklaven 90 Minuten in Stickstoffatmosphäre vorkondensiert, dann 4 Std. in einem offenen Polykondensationsgefäss im Stickstoffstrom weiterkondensiert und schliesslich 1 Std. im Hochvakuum nachkondensiert. Alle Polykondensationsschritte werden bei 250 °C durchgeführt.

Elementaranalyse des erhaltenen Polyamids :
berechnet : C 72,88 % H 10,57 % N 7,73 %
gefunden : C 70,20 % H 10,47 % N 7,45 %.
Endgruppengehalt : —COOH 0,16 m Aequiv./g ; —$NH_2$ 0,04 m Aequiv./g.

Glasumwandlungstemperatur Tg 45 °C [bestimmt im Differentialkalorimeter (DSC)] ; reduzierte Viskosität (0,5%ige Lösung in m-Kresol bei 25 °C) η red = 0,70 dl/g.

## Beispiel III

a) Herstellung eines Salzes aus Terephthalsäure (TPS) und 2,2-Dimethyl-11-isopropyl-1,11-diamino-undeca-4,8-dien (Undecadien-diamin = UDD) :

97,1 g Terephthalsäure werden in 2 400 ml Wasser und 600 ml Methanol suspendiert. Zur erhaltenen Suspension tropft man bei Rückflusstemperatur 148,9 g UDD zu, wobei eine homogene Lösung entsteht. Nach 60 Minuten Kochen lässt man die Lösung auf 0-5 °C abkühlen. Nach 24 Stunden wird vom gebildeten Salz abfiltriert und bei 80 °C im Vakuum getrocknet. Ausbeute 180 g (73,6 % d. Th.).

b) Herstellung eines Salzes aus Adipinsäure (AS) und UDD :

36,2 g Adipinsäure werden in 270 ml absolutem Aethanol bei 50 °C gelöst. Nach dem Abkühlen der Lösung werden 69,7 g UDD in 107 ml absolutem Aethanol zugegeben. Das Salz, das nach Abziehen von zwei Dritteln des Lösungsmittels und Abkühlen des Restes auf 0-5 °C ausfällt, wird abfiltriert und im Vakuum bei 20 °C getrocknet. Ausbeute 82,5 g (85,3 % d. Th.).

56,0 g des gemäss a) erhaltenen Salzes aus TPS und UDD sowie 14,0 g des obigen Salzes aus Adipinsäure und UDD werden wie in Beispiel II beschrieben polykondensiert, jedoch unter Weglassen der letzten Kondensationsstufe im Vakuum. Glasumwandlungstemperatur des Polyamids = 105 °C, η red (0,5 %ige Lösung in m-Kresol bei 25 °C) = 0,72 dl/g.

Beispiel IV

Eine 10 %ige Lösung des gemäss Beispiel II hergestellten Polyamids in Chloroform/Aethanol (1 : 1), die 5 Gew.% Cumolhydroperoxid enthält, wird mit Hilfe eines 50 µm-Rakels auf eine Kupfer-Leiterplatte gegossen. Die Beschichtung wird 3 Minuten bei 100 °C getrocknet, worauf die Schichtdicke ca. 5 µm beträgt. Eine Nachhärtung bei 150 °C während 90 Minuten in Stickstoffatmosphäre ergibt eine harte, transparente und glänzende Schicht mit guter Haftung auf dem Kupfer. Lässt man die so behandelte Platte 72 Stunden in 60 °C warmen Chloroform stehen, so wird das Polyamid nicht abgelöst, und die Haftung des Polyamids reicht aus, um eine Aetzung des darunterliegenden Kupfers zu verhindern. Nach einer Woche Lagerung in Wasser bei 20 °C behält die Beschichtung ihre Transparenz und gute Haftung auf dem Kupfer ; Wasseraufnahme unter 1 Gew.%.

Beispiel V

i) 2,5 g des gemäss Beispiel II erhaltenen Polyamids, 1,5 g Penta-erythritol-tetrakis-(3-mercaptopropionat) und 95 mg Thioxanthon werden in 10,8 ml Chloroform gelöst und mit einem 50 µm-Rakel auf eine 100 µm-dicke Polyesterfolie aufgetragen. Die Beschichtung wird 3 Minuten bei 100 °C getrocknet und mit einem 5 000 W Quecksilberhochdruckbrenner durch eine photographische Maske während 15 Sekunden belichtet (Abstand des Quecksilberhochdruckbrenners vom Vakuumtisch 70 cm). Nach 30 Sekunden Entwicklung in Chloroform erhält man ein gut aufgelöstes Negativ-Reliefbild.

ii) Das unter i) beschriebene Verfahren wird wiederholt unter Verwendung von 81,6 mg Benzophenon anstelle des Thioxanthons und einer Belichtungszeit von 60 Sekunden.

| Versuch | Belichtungszeit (Sekunden) | letzte abgebildete Stufe auf einem 21 step-sensitivity guide der Fa. Stouffer |
|---|---|---|
| i) | 15 | 8 |
| ii) | 60 | 8 |

**Ansprüche**

1. Verbindungen der Formel I

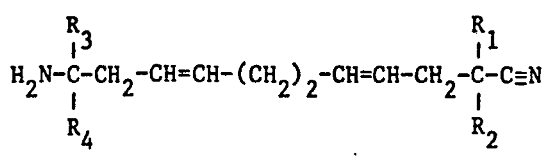

(I)

worin
$R_1$ Alkyl mit 1-12 C-Atomen,
$R_2$ Wasserstoff oder Alkyl mit 1-12 C-Atomen,
$R_3$ Alkyl mit 1-12 C-Atomen, Cycloalkyl mit 4-12 Ring-C-Atomen, Aralkyl mit 7 oder 8 C-Atomen, gegebenenfalls substituiertes Aryl oder, wenn $R_4$ Wasserstoff ist, auch —CH = CH-Alkyl oder —C(Alkyl) = CH-Alkyl mit je 1-4 C-Atomen in den Alkylgruppen und
$R_4$ Wasserstoff, Alkyl mit 1-12 C-Atomen, Cycloalkyl mit 4-12 Ring-C-Atomen, Aralkyl mit 7 oder 8 C-Atomen oder gegebenenfalls substituiertes Aryl darstellen oder
$R_1$ und $R_2$ und/oder $R_3$ und $R_4$ zusammen Alkylen mit 3-11 C-Atomen bedeuten.

2. Verbindungen der Formel I nach Anspruch 1, worin $R_1$ Alkyl mit 1-5 C-Atomen, $R_2$ Wasserstoff oder Alkyl mit 1-5 C-Atomen oder $R_1$ und $R_2$ zusammen Alkylen mit 4-7 C-Atomen, $R_3$ Alkyl mit 1-7 C-Atomen, Cycloalkyl mit 5-8 C-Atomen oder unsubstituiertes Phenyl oder, bei $R_4$ = H, auch $—C(C_2H_5) = CHCH_3$, und $R_4$ Wasserstoff oder Alkyl mit 1-5 C-Atomen bedeuten.

3. Verbindungen der Formel I nach Anspruch 2, worin $R_3$ Alkyl mit 1-5 C-Atomen, unsubstituiertes Phenyl oder, bei $R_4$ = H, $—C(C_2H_5) = CHCH_3$, und $R_4$ Wasserstoff oder Methyl bedeuten.

4. Verbindungen der Formel I nach Anspruch 1, worin $R_1$ Methyl oder Aethyl, $R_2$ Wasserstoff, Methyl oder Aethyl, $R_3$ Alkyl mit 1-5 C-Atomen oder unsubstituiertes Phenyl und $R_4$ Wasserstoff oder Methyl bedeuten.

5. Verbindung der Formel I nach Anspruch 1, worin $R_1$ und $R_2$ Methyl, $R_3$ Isopropyl und $R_4$ Wasserstoff darstellen.

6. Verfahren zur Herstellung von Verbindungen der Formel I nach Anspruch 1, dadurch gekennzeichnet, dass man Verbindungen der Formel II

$$H_2N-\overset{R_3}{\underset{R_4}{C}}-CH_2-CH=CH-(CH_2)_2-CH=CH-CH_2-\overset{R_1}{\underset{R_2}{C}}-CH=NOH \qquad (II)$$

worin $R_1$, $R_2$, $R_3$ und $R_4$ die unter Formel I angegebene Bedeutung haben, dehydratisiert.

## Claims

1. A compound of the formula I

$$H_2N-\overset{R_3}{\underset{R_4}{C}}-CH_2-CH=CH-(CH_2)_2-CH=CH-CH_2-\overset{R_1}{\underset{R_2}{C}}-C\equiv N$$

in which

$R_1$ is alkyl having 1-12 C atoms,

$R_2$ is hydrogen or alkyl having 1-12 C atoms,

$R_3$ is alkyl having 1-12 C atoms, cycloalkyl having 4-12 ring C atoms, aralkyl having 7 or 8 C atoms, substituted or unsubstituted aryl or, if $R_4$ is hydrogen, —CH = CH-alkyl or —C(alkyl) = CH-alkyl each having 1-4 C atoms in the alkyl groups, and

$R_4$ is hydrogen, alkyl having 1-12 C atoms, cycloalkyl having 4-12 ring C atoms, aralkyl having 7 or 8 C atoms or substituted or unsubstituted aryl, or

$R_1$ and $R_2$ and/or $R_3$ and $R_4$ together are alkylene having 3-11 C atoms.

2. A compound of the formula I according to claim 1 in which $R_1$ is alkyl having 1-5 C atoms, $R_2$ is hydrogen or alkyl having 1-5 C atoms, or $R_1$ and $R_2$ together are alkylene having 4-7 C atoms, $R_3$ is alkyl having 1-7 C atoms, cycloalkyl having 5-8 C atoms, unsubstituted phenyl or, if $R_4$ = H, $—C(C_2H_5) = CHCH_3$, and $R_4$ is hydrogen or alkyl having 1-5 C atoms.

3. A compound of the formula I according to claim 2 in which $R_3$ is alkyl having 1-5 C atoms, unsubstituted phenyl or, if $R_4$ = H, $—C(C_2H_5) = CHCH_3$, and $R_4$ is hydrogen or methyl.

4. A compound of the formula I according to claim 1 in which $R_1$ is methyl or ethyl, $R_2$ is hydrogen, methyl or ethyl, $R_3$ is alkyl having 1-5 C atoms or unsubstituted phenyl, and $R_4$ is hydrogen or methyl.

5. The compound of the formula I according to claim 1 in which $R_1$ and $R_2$ are methyl, $R_3$ is isopropyl and $R_4$ is hydrogen.

6. A process for the preparation of a compound of the formula I according to claim 1, which comprises dehydrating a compound of the formula II

$$H_2N-\overset{R_3}{\underset{R_4}{C}}-CH_2-CH=CH-(CH_2)_2-CH=CH-CH_2-\overset{R_1}{\underset{R_2}{C}}-CH=NOH \qquad (II)$$

in which $R_1$, $R_2$, $R_3$ and $R_4$ are as defined under the formula I.

## Revendications

1. Composés de formule I

$$H_2N-\overset{R_3}{\underset{R_4}{C}}-CH_2-CH=CH-(CH_2)_2-CH=CH-CH_2-\overset{R_1}{\underset{R_2}{C}}-C\equiv N \qquad (I)$$

dans laquelle

$R_1$ est un radical alkyle à 1 à 12 atomes de carbone,

$R_2$ est l'hydrogène ou un radical alkyle à 1 à 12 atomes de carbone,

$R_3$ est un radical alkyle à 1 à 12 atomes de carbone, cycloalkyle à 4 à 12 atomes de carbone dans le cycle, aralkyle à 7 ou 8 atomes de carbone, aryle éventuellement substitué ou bien, quand $R_4$ est l'hydrogène, aussi —CH = CH-alkyle ou —C(alkyl) = CH-alkyle avec 1 à 4 atomes de carbone dans chaque groupe alkyle, et

$R_4$ est l'hydrogène, un radical alkyle à 1-12 atomes de carbone, cycloalkyle à 4-12 atomes de carbone dans le cycle, aralkyle à 7 ou 8 atomes de carbone ou aryle éventuellement substitué, ou bien

$R_1$ et $R_2$ et/ou $R_3$ et $R_4$ forment ensemble un radical alkylène à 3-11 atomes de carbone.

2. Composés de formule I selon la revendication 1, où $R_1$ est un radical alkyle à 1-5 atomes de carbone, $R_2$ est l'hydrogène ou un radical alkyle à 1-5 atomes de carbone ou bien $R_1$ et $R_2$ forment ensemble un radical alkylène à 4-7 atomes de carbone, $R_3$ est un radical alkyle à 1-7 atomes de carbone, cycloalkyle à 5-8 atomes de carbone ou phényle substitué ou bien aussi, pour $R_4$ = H, —$C(C_2H_5)$ = $CHCH_3$, et $R_4$ est l'hydrogène ou un radical alkyle à 1-5 atomes de carbone.

3. Composés de formule I selon la revendication 2, où $R_3$ est un radical alkyle à 1-5 atomes de carbone, phényle non substitué ou bien, pour $R_4$ = H, —$C(C_2H_5)$ = $CHCH_3$, et $R_4$ est l'hydrogène ou le radical méthyle.

4. Composés de formule I selon la revendication 1, où $R_1$ est le radical méthyle ou éthyle, $R_2$ est l'hydrogène, le radical méthyle ou éthyle, $R_3$ est un radical alkyle à 1-5 atomes de carbone ou phényle non-substitué, et $R_4$ est l'hydrogène ou le radical méthyle.

5. Composé de formule I selon la revendication 1, où $R_1$ et $R_2$ sont le radical méthyle, $R_3$ est le radical isopropyle et $R_4$ est l'hydrogène.

6. Procédé de préparation de composés de formule I selon la revendication 1, caractérisé en ce qu'on déshydrate des composés de formule II

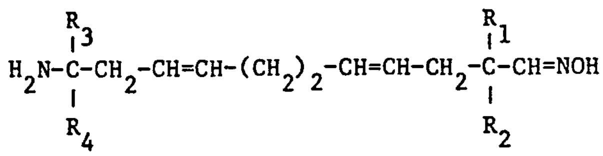

(II)

où $R_1$, $R_2$, $R_3$ et $R_4$ ont la signification donnée à propos de la formule I.